# EUROPÄISCHE PATENTSCHRIFT

(11) **EP 0 638 074 B1**
(45) Veröffentlichungstag und Bekanntmachung des Hinweises auf die Patenterteilung: **31.03.1999**
(21) Anmeldenummer: 93911471.6
(22) Anmeldetag: 16.04.1993
(51) Int. Cl.: C07D 261/18, C07D 413/12, A01N 43/80

(54) **ISOXAZOLCARBONSÄUREAMIDE, VERFAHREN ZU IHRER HERSTELLUNG UND IHRE VERWENDUNG**
ISOXAZOLE CARBOXYLIC ACID AMIDES, PROCESS FOR PRODUCING THEM AND THEIR USE
AMIDES DE L'ACIDE ISOXAZOLCARBOXYLIQUE, LEUR PROCEDE DE FABRICATION ET LEUR UTILISATION

(30) Priorität: 29.04.1992 DE 4214010
(43) Veröffentlichungstag der Anmeldung: 15.02.1995
(73) Patentinhaber: BASF Aktiengesellschaft, 67063 Ludwigshafen (DE)
(72) Erfinder: MAYWALD, Volker, D-6700 Ludwigshafen (DE); KUEKENHOEHNER, Thomas, D-6737 Boehl-Iggelheim (DE); MUENSTER, Peter, D-6823 Neulussheim (DE); VON DEYN, Wolfgang, D-6730 Neustadt (DE); WALTER, Helmut, D-6719 Obrigheim (DE); GERBER, Matthias, D-6703 Limburgerhof (DE); WESTPHALEN, Karl-Otto, D-6720 Speyer (DE); KARDORFF, Uwe, D-6800 Mannheim 1 (DE)
(86) Internationale Anmeldenummer: EP9300918
(87) Internationale Veröffentlichungsnummer: WO9322295

(56) Entgegenhaltungen:
- EP-A- 0 418 667

## Beschreibung

Die vorliegende Erfindung betrifft Isoxazolcarbonsäureamide der Formel I 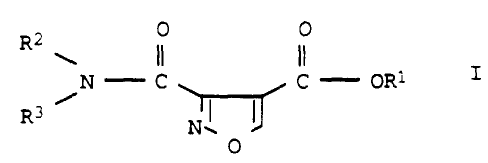 in der die Substituenten die folgende Bedeutung haben:
R¹
   (a) eine C₁-C₆-Alkylgruppe, welche ein bis fünf Halogenatome und/oder bis zu drei Hydroxy- und/oder C₁-C₄-Alkoxygruppen und/oder einen der folgenden Reste tragen kann:
      - Cyano;
      - C₁-C₄-Alkoxy-C₂-C₄-alkoxy;
      - C₁-C₃-Alkylthio;
      - C₁-C₃-Alkylamino, Di-(C₁-C₃-alkyl)amino;
      - C₃-C₆-Cycloalkylamino oder Di-(C₃-C₆-cycloalkyl)amino;
      - Tri(C₁-C₄-alkyl)silyl;
      - Carboxyl, C₁-C₃-Alkoxycarbonyl oder C₁-C₃-Alkoxycarbonyl-C₁-C₃-alkoxy, Di(C₁-C₃-alkyl)aminocarbonyl
      - C₁-C₆-Alkaniminoxy oder C₅-C₆-Cycloalkaniminoxy;
      - einen 5- oder 6-gliedrigen gesättigten oder ungesättigten heterocyclischen Rest mit jeweils bis zu 3 Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, C₁-C₃-Alkyl oder C₁-C₃-Alkoxy;
      - C₃-C₆-Cycloalkyl;
      - Phenyl, das noch bis zu drei der folgenden Substituenten tragen kann: Halogen, Nitro, Cyano, C₁-C₃-Alkyl, partiell oder vollständig halogeniertes C₁-C₃-Alkyl, C₁-C₃-Alkoxy oder partiell oder vollständig halogeniertes C₁-C₃-Alkoxy;
      - einen Rest -CR¹⁰=N-R¹¹, wobei R¹⁰ und R¹¹ die folgende Bedeutung haben:
         - R¹⁰: Wasserstoff oder C₁-C₆-Alkyl und
         - R¹¹: C₁-C₆-Alkoxy, C₃-C₆-Alkenyloxy oder C₃-C₆-Alkinyloxy, die jeweils bis zu drei Halogenatome und/oder einen Phenylrest mit gewünschtenfalls bis zu drei der folgenden Reste tragen können: Halogen, Nitro, Cyano, C₁-C₃-Alkyl oder C₁-C₃-Alkoxy;
         C₁-C₆-Alkylamino, Di-(C₁-C₆-alkyl)amino oder Phenylamino, wobei der Phenylrest zusätzlich bis zu drei der folgenden Reste tragen kann: Halogen, Nitro, Cyano, C₁-C₃-Alkyl oder C₁-C₃-Alkoxy;
   (b) C₃-C₈-Cycloalkyl, das einen bis fünf der folgenden Reste tragen kann: Halogen, C₁-C₄-Alkyl, C₁-C₄-Alkoxy partiell oder vollständig halogeniertes C₁-C₄-Alkyl oder partiell oder vollständig halogeniertes C₁-C₄-Alkoxy;
   (c) gegebenenfalls durch C3-C6-Alkinyl, Halogen, C₁-C₄-Alkoxy oder Phenyl substituiertes verzweigtes oder unverzweigtes C₃-C₆-Alkenyl,
   (d) C₅-C₆-Cycloalkenyl,
   (e) gegebenenfalls durch Halogen, C₁-C₄-Alkoxy oder Phenyl substituiertes verzweiges oder unverzweigtes C₃-C₆-Alkinyl, wobei der Phenylrest seinerseits eine bis drei der folgenden Gruppen tragen kann: Halogen, Nitro, Cyano, C₁-C₄-Alkyl, partiell oder vollständig halogeniertes C₁-C₄-Alkyl oder C₁-C₄-Alkoxy, partiell oder vollständig halogeniertes C₁-C₄-Alkoxy;
   (f) Phenyl, das eine bis drei der folgenden Gruppen tragen kann:
      - Halogen, Nitro, Cyano, C₁-C₄-Alkyl, partiell oder vollständig halogeniertes C₁-C₄-Alkyl, C₁-C₄-Alkoxy oder partiell oder vollständig halogeniertes C₁-C₄-Alkoxy;
   (g) N-Phthalimido, Tetrahydrophthalimido, N-Succinimido oder Maleinimido;
R²
   Wasserstoff oder C₁-C₆-Alkyl;
R³
   (a) eine C₁-C₆-Alkylgruppe, die einen bis drei der folgenden Reste tragen kann: Halogen, Cyano, C₁-C₄-Alkoxy, C₃-C₈-Cycloalkyl oder Phenyl, wobei der Phenylring seinerseits einen bis drei der folgenden Reste tragen kann: Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy,
   (b) eine C₃-C₈-Cycloalkylgruppe, die einen bis drei der folgenden Reste tragen kann: Halogen, C₁-C₆-Alkyl, partiell oder vollständig halogeniertes C₁-C₆-Alkyl, C₁-C₄-Alkoxy oder partiell oder vollständig halogeniertes C₁-C₄-Alkoxy;
   (c) eine verzweigte oder unverzweigte C₃-C₈-Alkenyl- oder verzweigte oder unverzweigte C₃-C₈-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro;
      C₁-C₄-Alkoxy, C₁-C₄-Halogenalkoxy, C₁-C₄-Alkylthio, C₁-C₄-Halogenalkylthio, Halogen, Cyano oder Nitro;
   (d) eine Phenylgruppe, die eine bis vier der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Nitro, Cyano, Formyl, C₁-C₄-Alkanoyl, C₁-C₄-Halogenalkanoyl oder C₁-C₄-Alkoxycarbonyl;
   sowie die pflanzenverträglichen Salze derjenigen Verbindungen, in denen R² für Wasserstoff steht.

Des weiteren betrifft die Erfindung herbizide Mittel, die diese Verbindungen enthalten, sowie Verfahren zur Herstellung dieser Verbindungen und ein allgemeines Verfahren zur Herstellung von Isoxazolcarbonsäurederivaten der Formel I', 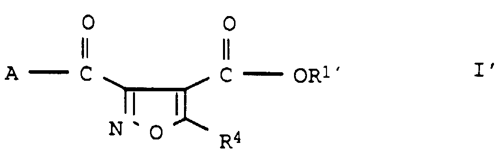 in der die Substituenten die folgende Bedeutung haben:
- R^{1'}: ggf. substituiertes Alkyl, Cycloalkyl, Phenyl, Alkenyl oder Alkinyl;
- A: eine Gruppe NR^{2'}R^{3'} oder eine Gruppe OR⁵;
- R^{2'}: Wasserstoff oder Alkyl;
- R^{3'}: ggf. substituiertes Alkyl, Cycloalkyl, Phenyl, Alkenyl, Alkinyl oder Aryl;
oder R^{2'} und R^{3'} gemeinsam -CH₂CH₂CH₂CH₂- oder -CH₂CH₂CH₂CH₂CH₂-, wobei in diesen Ketten eine CH₂-Gruppe durch Sauerstoff, Schwefel oder NCH₃ ersetzt sein kann;
- R⁵: ggf. substituiertes Alkyl, Cycloalkyl, Phenyl, Alkenyl oder Alkinyl;
- R⁴: Wasserstoff oder Alkyl,
welches dadurch gekennzeichnet ist, daß man ein Enamin der Formel II 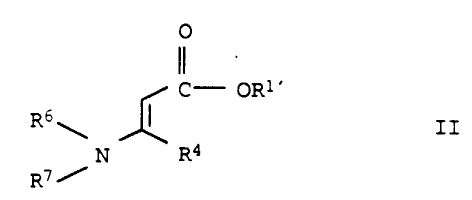 in der R⁶ und R⁷ für Wasserstoff oder C₁-C₄-Alkyl stehen oder gemeinsam -CH₂CH₂CH₂CH₂- oder -CH₂CH₂CH₂CH₂CH₂- bedeuten, wobei in diesen Ketten eine CH₂-Gruppe durch Sauerstoff, Schwefel oder NCH₃ ersetzt sein kann, mit einem Hydroxamsäurechlorid der Formel III 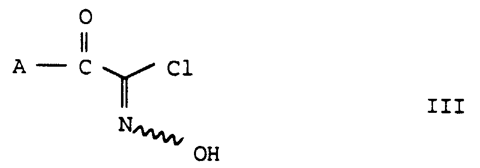 umsetzt.

Aus EP-A 418,667 sind Isoxazolcarbonsäureamide als herbizide Wirkstoffe bekannt, deren allgemeine Formel die Verbindungen I umfaßt. Beispiele für die spezielle Struktur I sind in dieser Schrift jedoch nicht gegeben.

Aufgabe der vorliegenden Erfindung waren neue herbizide Wirkstoffe mit verbesserten Eigenschaften.

Demgemäß wurden die eingangs definierten Verbindungen I, herbizide Mittel, die diese Verbindungen I enthalten, sowie Verfahren zu deren Herstellung und Anwendung gefunden.

Außerdem wurde ein verbessertes Verfahren zur Herstellung der Verbindungen der allgemeinen Formel I' gefunden.

Die erfindungsgemäßen Verbindungen zeichnen sich gegenüber aus dem Stand der Technik bekannten Verbindungen dadurch aus, daß kein Substituent in 5-Position des Isoxazolrings vorliegt. Wesentlich für die vorteilhafte herbizide Wirkung ist insbesondere die Kombination von Wasserstoff in 5-Position mit einer Estergruppierung in 4-Position, wobei die Art des Esters keine besondere Bedeutung spielt, d.h. der Rest R¹ ist sehr breit variabel.

Aus dem US-Patent Nr. 3 699 117 ist bekannt, daß 5-Bisalkoxymethyl-isoxazol-3,4-dicarbonsäureester durch Umsetzung von α-Chloroximinoacetaten mit Enaminen von γ,γ-Dialkoxyacetessigsäureestern zugänglich sind.

In der Formel II stehen die Reste R⁶ und R⁷ für Wasserstoff oder C₁-C₄-Alkyl wie Methyl, Ethyl, Propyl, 1-Methylethyl, Butyl, 1-Methylpropyl, 2-Methylpropyl und 1,1-Dimethylethyl, vorzugsweise 1-Methylethyl, 1-Methylpropyl und 1,1-Dimethylethyl, oder die Reste R⁶ und R⁷ stehen gemeinsam für-CH₂CH₂CH₂CH₂- oder -CH₂CH₂CH₂CH₂CH₂-, wobei in diesen Ketten eine CH₂-Gruppe durch Sauerstoff, Schwefel oder NCH₃ ersetzt sein kann, beispielsweise -CH₂OCH₂CH₂-, -CH₂SCH₂CH₂-, -CH₂N(CH₃)CH₂CH₂-, -CH₂CH₂OCH₂CH₂-, -CH₂CH₂SCH₂CH₂-, -CH₂CH₂N(CH₃)CH₂CH₂-, -CH₂OCH₂CH₂CH₂-, -CH₂SCH₂CH₂CH₂- und-CH₂N(CH₃)CH₂CH₂CH₂-, vorzugsweise -CH₂CH₂CH₂CH₂-, -CH₂CH₂CH₂CH₂CH₂-, -CH₂CH₂OCH₂CH₂- oder -CH₂CH₂SCH₂CH₂-.

Diese Umsetzung wird üblicherweise bei Temperaturen von 10°C bis 120°C, vorzugsweise 25°C bis 70°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran sowie Dimethylsulfoxid und Dimethylformamid, besonders bevorzugt Ether wie vorstehend genannt. Es können auch Gemische der genannten Lösungsmittel verwendet werden.

Die Umsetzung kann in Gegenwart oder Abwesenheit einer HCl bindenden Base, beispielsweise eines tertiären Amins, erfolgen. Gemäß einer bevorzugten Ausführungsform des Verfahrens wird auf den Zusatz einer Base verzichtet.

Die Edukte werden im allgemeinen in äquimolaren Mengen miteinander umgesetzt. Es kann jedoch für die Ausbeute vorteilhaft sein, eine der Komponenten im Überschuß einzusetzen..

Die für das erfindungsgemäße Verfahren benötigten Enamine II sind entweder kommerziell erhältlich oder nach allgemein bekannten Verfahren herstellbar, z.B. wie in Chem. Ber. 99, S. 2526-2545, 1966 (C.A. 65: 15316 d) beschrieben.

Die Hydroxamsäurechloride III sind literaturbekannt (vgl. z.B. DE-A1-28 17 838; J. Org. Chem., Vol. 48, No. 3, 1983; US 3,557,190) oder beispielsweise nach dem in DE-A 19 63 061 beschriebenen Verfahren, ausgehend von Acetoacetamiden herstellbar. Nach diesem allgemeinen Verfahren lassen sich prinzipiell auch Hydroxamsäurechloride, die bisher nicht in der Literatur beschrieben sind, herstellen.

Das vorstehend geschilderte Verfahren eignet sich insbesondere zur Herstellung von Verbindungen der allgemeinen Formel I', wobei die Substituenten für die folgenden Reste stehen:
R^{1'}
   ggf. substituiertes Alkyl, vorzugsweise unverzweigtes oder verzweigtes niedermolekulares Alkyl wie C₁-C₆-Alkyl, z.B. Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl, insbesondere Methyl und Ethyl;
   ggf. substituiertes Cycloalkyl, vorzugsweise C₃-C₈-Cycloalkyl wie Cyclopropyl, Cyclobutyl, Cyclopentyl, Cyclohexyl, Cycloheptyl und Cyclooctyl, insbesondere Cyclopropyl, Cyclopentyl und Cyclohexyl;
   ggf. substituiertes Alkenyl, vorzugsweise unverzweigtes oder verzweigtes C₃-C₆-Alkenyl wie 2-Butenyl, 3-Butenyl, 1-Methyl-2-propenyl, 2-Methyl-2-propenyl, 2-Pentenyl, 3-Pentenyl, 4-Pentenyl,
   1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, l-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, l-Ethyl-1-methyl-2-propenyl und l-Ethyl-2-methyl-2-propenyl, insbesondere 2-Propenyl;
   ggf. substituiertes Alkinyl, vorzugsweise unverzweigtes oder verzweigtes C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und l-Ethyl-l-methyl-2-propinyl, insbesondere 2-Propinyl.

In den vorstehend genannten Resten können ein Teil oder alle der Wasserstoffatome durch Halogenatome wie Fluor, Chlor, Brom und Jod, vorzugsweise Fluor und/oder Chlor ersetzt sein. Außerdem können diese Reste ein bis drei zusätzliche, unter den Umsetzungsbedingungen stabile Substituenten tragen, z.B.:
- ggf. substituiertes Phenyl,
- C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
- C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy und Ethoxy;
- C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
- C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio;
- C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio.

Die Cycloalkylreste können neben den vorstehend genannten Gruppen außerdem ein bis drei der folgenden Reste tragen:
- C₁-C₄-Alkyl wie Methyl, Ethyl, Propyl, 1-Methylethyl, Butyl, 1-Methylpropyl, 2-Methylpropyl und 1,1-Dimethylethyl.

Der Phenylrest seinerseits kann neben den vorstehend genannten Halogenatomen ein bis drei der folgenden Reste tragen: die vorstehend genannten C₁-C₄-Halogenalkyl-, C₁-C₄-Alkoxy-, C₁-C₄-Halogenalkoxy-, C₁-C₄-Alkylthio- und C₁-C₄-Halogenalkylthio-Gruppen und/oder C₁-C₄-Alkyl wie Methyl, Ethyl, Propyl, 1-Methylethyl, Butyl, 1-Methylpropyl, 2-Methylpropyl und 1,1-Dimethylethyl.
A
   steht für eine Gruppe NR^{2'}R^{3'} oder eine Gruppe OR⁵.

Hierbei steht R^{2'} für Wasserstoff oder für Alkyl wie vorstehend im allgemeinen und im besonderen genannt.
R^{3'} steht für ggf. substituiertes Alkyl, Cycloalkyl, Alkenyl oder Alkinyl wie vorstehend im allgemeinen und im besonderen genannt oder für ggf. substituiertes Aryl wie Phenyl, 1-Naphthyl und 2-Naphthyl, wobei in diesen aromatischen Resten ein Teil oder alle Wasserstoffatome durch Halogenatome wie vorstehend im allgemeinen und im besonderen genannt ersetzt sein können und wobei diese aromatischen Gruppen außerdem ein bis drei der folgenden Reste tragen können:
- C₁-C₄-Alkyl wie Methyl, Ethyl, Propyl, 1-Methylethyl, Butyl, 1-Methylpropyl, 2-Methylpropyl und 1,1-Dimethylethyl;
- C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
- C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy;
- C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
- C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio;
- C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio.

Außerdem können die Reste R^{2'} und R^{3'} gemeinsam für -CH₂CH₂CH₂CH₂- oder -CH₂CH₂CH₂CH₂CH₂-, stehen, wobei in diesen Ketten eine CH₂-Gruppe durch Sauerstoff, Schwefel oder NCH₃ ersetzt sein kann, z.B.: -CH₂OCH₂CH₂-, -CH₂SCH₂CH₂-, -CH₂N(CH₃)CH₂CH₂-, -CH₂CH₂OCH₂CH₂-, -CH₂CH₂SCH₂CH₂-, -CH₂CH₂NHCH₂CH₂-, -CH₂CH₂N(CH₃)CH₂CH₂-, -CH₂OCH₂CH₂CH₂-, -CH₂SCH₂CH₂CH₂-, und -CH₂N(CH₃)CH₂CH₂CH₂-.

R⁵ steht für ggf. substituiertes Alkyl, Cycloalkyl, Phenyl, Alkenyl oder Alkinyl wie vorstehend bei R^{1'} im allgemeinen und im besonderen genannt.

R⁴ steht für Wasserstoff oder niedermolekulares unverzweigtes Alkyl, besonders C₁-C₆-Alkyl wie Methyl, Ethyl, Propyl, Butyl, Pentyl und Hexyl. Vorzugsweise bedeutet R⁴ Wasserstoff.

Neben dem vorstehend geschilderten Verfahren eignen sich auch herkömmliche, aus der Literatur bekannte Verfahren zur Herstellung der Verbindungen I. Besonders bevorzugt erhält man die Verbindungen I ausgehend von Isoxazol-3-carbonsäureamid-4-alkylestern, z.B. -4-C₁-C₆-Alkylestern wie Methyl oder Ethylestern, durch Umesterung gemäß der folgenden Reaktionsgleichung, indem man zunächst den Alkylester in an sich bekannter Weise (vgl. J. March, Advanced Organic Chemistry, 2nd Edition, McGraw-Hill International Book Company, 1977, Seite 349 ff.) in Gegenwart einer Base spaltet und die so erhaltene Saure IV anschließend mit einem Alkohol V verestert:

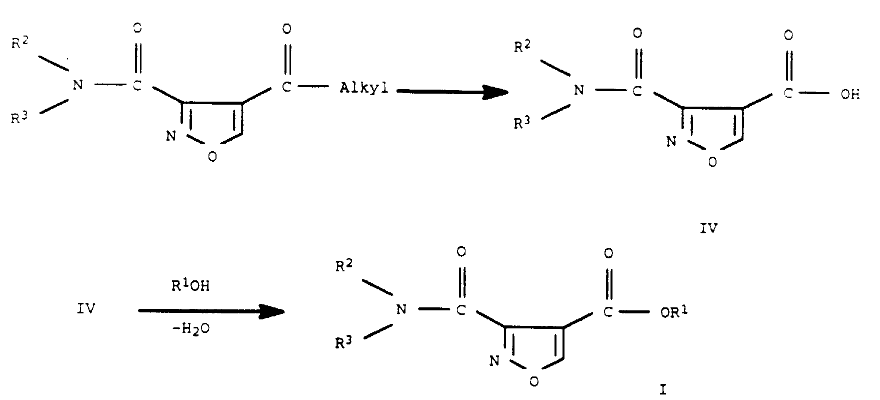

Diese Spaltung des Alkylesters zu IV wird üblicherweise bei Temperaturen von -10°C bis 50°C, vorzugsweise 0°C bis 30°C in Wasser oder einem organischen Lösungsmittel durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol sowie Dimethylsulfoxid, Dimethylformamid und Wasser, besonders bevorzugt Alkohole wie vorstehend genannt oder Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallcarbonate wie Lithiumcarbonat, Natriumcarbonat und Calziumcarbonat sowie Alkalimetallhydrogencarbonate wie Natriumhydrogencarbonat in Betracht.

Besonders bevorzugt werden Alkalimetall- und Erdalkalimetallhydroxide Natriumhydroxid und Kaliumhydroxid.

Die Basen werden im allgemeinen äquimolar oder im Überschuß verwendet.

Für den Fall, daß sich die Base im verwendeten Lösungsmittel nicht in ausreichendem Maß löst (zweiphasige Reaktionsführung) kann es im Hinblick auf die Umsetzungsgeschwindigkeit und die Ausbeute vorteilhaft sein, zusätzlich der Reaktionsmischung einen Phasentransferkatalysator, z.B. einen Kronenether, zuzusetzen.

Die Veresterung der freien Säure IV mit einem Alkohol V zur Verbindung I kann nach den allgemein üblichen Veresterungsmethoden durchgeführt werden (vgl. J. March, Advanced Organic Chemistry, 2nd Edition, McGraw-Hill International Book Company, 1977, Seite 361 ff., 363 ff. und die dort zitierte Literatur).

Üblicherweise überführt man hierbei die Säure IV zunächst in eine aktivierte Form, welche anschließend bei Temperaturen von 0°C bis 100°C, vorzugsweise 25°C bis 70°C mit dem Alkohol V zur Reaktion gebracht wird.

Als Lösungsmittel verwendet man zweckmäßigerweise aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Ketone wie Aceton, Methylethylketon, Diethylketon und tert.-Butylmethylketon, Ester von organische Säuren wie Essigsäureethylester sowie Dimethylsulfoxid und Dimethylformamid. Es können auch Gemische der genannten Lösungsmittel verwendet werden.

Zur Aktivierung der Carbonsäure IV eignen sich beispielsweise Halogenverbindungen wie insbesondere Thionylchlorid, Oxalylchlorid und Phosgen oder wasserentziehende Mittel wie Dicyclohexylcarbodiimid, Carbonyldiimidazol oder Säureanhydride wie Essigsäureanhydrid, Propanphosphonsäureanhydrid und 1-Methyl-2-halogenpyridiniumiodid (Chem. Lett., 1045 (1975); Chem. Lett., 13 (1976); Chem. Lett., 49 (1976)).

Im Hinblick auf die bestimmungsgemäße Verwendung der Verbindungen I als Herbizide sind insbesondere solche Substanzen bevorzugt, in denen die Substituenten die folgende Bedeutung haben:
R¹
   (a) C₁-C₆-Alkyl, bevorzugt C₁-C₄-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise C₁-C₄-Alkyl, insbesondere Methyl oder Ethyl oder tert.-Butyl, welches ein bis fünf Halogenatome wie Fluor, Chlor, Brom oder Jod, insbesondere Fluor und Chlor, und/oder bis zu drei Hydroxy- und/oder C₁-C₄-Alkoxygruppen wie Methoxy,Ethoxy, n-Propoxy, Iso-propoxy, n-Butoxy und tert.-Butoxy, und/oder einen der folgenden Reste tragen kann:
      - Cyano,
      - C₁-C₄-Alkoxy-C₂-C₄-alkoxy, insbesondere Methoxy-ethoxy, Ethoxy-ethoxy und Propoxy-ethoxy,
      - C₁-C₃-Alkylthio, insbesondere Methylthio und Ethylthio,
      - C₁-C₃-Alkylamino wie Methylamino, Ethylamino und Isopropylamino,
      - Di-(C₁-C₃-alkyl)amino wie Dimethylamino, Diethylamino, Dipropylamino, Di(1-methylethyl)amino und Methylethylamino,
      - C₃-C₆-Cycloalkylamino oder Di-(C₃-C₆-cycloalkyl)amino wie Cyclopropylamino oder Dicyclopropylamino,
      - Tri(C₁-C₄-alkyl)silyl wie Trimethylsilyl oder Triethylsilyl,
      - Carboxyl
      - C₁-C₃-Alkoxycarbonyl wie Methoxycarbonyl und Isopropylcarbonyl,
      - C₁-C₃-Alkoxycarbonyl-C₁-C₃-alkoxy wie Methoxycarbonylmethoxy,
      - Di-(C₁-C₃-alkyl)aminocarbonyl wie Di-isopropylaminocarbonyl,
      - C₁-C₆-Alkaniminoxy wie 2-Propaniminoxy oder C₅-C₆-Cycloalkaniminoxy wie Cyclopentaniminoxy und Cyclohexaniminoxy,
      - einen 5- oder 6-gliedrigen gesättigten oder ungesättigten heterocyclischen Rest mit jeweils 1 bis 3 Heteroatomen, ausgewählt aus der Gruppe Sauerstoff, Schwefel und Stickstoff, wobei zwei Sauerstoff- oder Schwefelatome oder ein Sauerstoff und ein Schwefelatom nicht direkt benachbart sein können, insbesondere Tetrahydrofuran-2-yl, Tetrahydrofuran-3-yl, Tetrahydrothien-2-yl, Tetrahydrothien-3-yl, Tetrahydropyran-2-yl, Tetrahydropyran-3-yl, Tetrahydro-pyran-4-yl, Pyrrolidin-2-yl, Pyrrolidin-3-yl, Furan-2-yl, Furan-3-yl, Thien-2-yl, Thien-3-yl, Pyrrol-2-yl, Pyrrol-3-yl, Isoxazol-3-yl, Isoxazol-4-yl, Isoxazol-5-yl, Isothiazol-3-yl, Isothiazol-4-yl, Isothiazol-5-yl, Oxazol-2-yl, Oxazol-4-yl, Oxazol-5-yl, Thiazol-2-yl, Thiazol-4-yl, Thiazol-5-yl, Imidazol-2-yl, Imidazol-4-yl, Imidazol-5-yl, Pyrazol-1-yl, Pyrazol-3-yl, Pyrazol-4-yl, Pyrazol-5-yl, 1,2,3-Triazol-1-yl, 1,2,3-Triazol-4-yl, 1,2,3-Triazol-5-yl, 1,2,4-Triazol-1-yl, 1,2,4-Triazol-3-yl, 1,2,4-Triazol-5-yl, 1,2,3-Thiadi-azol-4-yl, 1,2,3-Thiadiazol-5-yl, 1,2,4-Thiadiazol-3-yl-, 1,2,4-Thiadiazol-5-yl, 1,2,5-Thiadiazol-3-yl, 1,2,5-Thiadiazol-4-yl, 1,3,4-Thiadiazol-2-yl, 1,3,4-Thiadiazol-5-yl, 1,2,3-Oxadiazol-3-yl, 1,2,3-Oxadiazol-5-yl, 1,2,4-Oxadiazol-3-yl, 1,2,4-Oxadiazol-5-yl, 1,2,5-Oxadiazol-3-yl, 1,2,5-Oxadiazol-4-yl, 1,3,4-Oxadiazol-2-yl, 1,3,4-Oxadiazol-5-yl, Pyrid-2-yl, Pyrid-3-yl, Pyrid-4-yl, Pyrimid-2-yl, Pyrimid-4-yl und Pyrimid-5-yl, wobei die Heterocyclen noch einen oder zwei der folgenden Substituenten tragen können: Halogen wie Fluor, Chlor, Brom und Jod, insbesondere Chlor und Brom und/ oder C₁-C₃-Alkyl wie Methyl, Ethyl, n-Propyl und iso-Propyl, C₁-C₃-Alkoxy wie Methoxy, Ethoxy, n-Propoxy und iso-Propoxy,
      - die Phenylgruppe, die noch einen bis drei der folgenden Substituenten tragen kann: Halogen wie Fluor, Chor, Nitro, Brom und Iod, insbesondere Fluor und Chlor, Nitro, Cyano C₁-C₃-Alkyl wie Methyl oder Isopropyl, partiell oder vollständig halogeniertes C₁-C₃-Alkyl wie Trifluormethyl, 1,1,2,2-Tetrafluorethyl und Trichlormethyl, C₁-C₃-Alkoxy wie Methoxy und Isopropoxy, und/oder partiell oder vollständig halogeniertes C₁-C₃-Alkoxy, insbesondere Trifluormethoxy;
      - einen Rest -CR¹⁰=N-R¹¹ mit
         - R¹⁰: Wasserstoff oder verzweigstes oder unverzweigstes C₁-C₆-Alkyl, insbesondere C₁-C₄-Alkyl wie Methyl, Ethyl, n-Propyl, iso-Propyl, n-Butyl und tert.-Butyl;
         - R¹¹: C₁-C₆-Alkoxy, C₃-C₆-Alkenyloxy oder C₃-C₆-Alkinyloxy, insbesondere C₁-C₄-Alkoxy wie Methoxy, Ethoxy, n-Propoxy, iso-Propoxy, n-Butoxy und tert.-Butoxy, sowie Prop-2-enyloxy, But-2-enyloxy, Prop-2-inyloxy und But-2-inyloxy, wobei diese Substituenten noch ein bis drei Halogenatome wie Fluor, Chlor, Brom und Iod, insbesondere Fluor und Chlor, und/oder einen Phenylrest, der unstubstituiert oder ein- bis dreifach durch Halogen wie vorstehend genannt, Nitro, Cyano, C₁-C₃-Alkyl wie Methyl, Ethyl, n-Propyl und iso-Propyl und/oder C₁-C₃-Alkoxy wie Methoxy, Ethoxy, n-Propoxy und iso-Propoxy substituiert sein kann, tragen können; Phenoxy, das noch einen bis drei der folgenden Substituenten tragen kann: Nitro, Cyano, Halogen wie vorstehend genannt, C₁-C₃-Alkyl wie vorstehend genannt und/oder C₁-C₃-Alkoxy wie vorstehen genannt;
         verzweigtes oder unverzweigtes C₁-C₆-Alkylamino, Di(C₁-C₆)-alkylamino oder Phenylamino, wobei der Aromat zusätzlich ein- bis dreifach durch Nitro, Cyano, Halogen wie vorstehend genannt, C₁-C₃-Alkyl wie vorstehend genannt und/oder C₁-C₃-Alkoxy wie vorstehend genannt, substituiert sein kann;
   (b) C₃-C₈-Cycloalkyl, bevorzugt C₃-C₆-Cycloalkyl wie Cyclopropyl, Cyclopentyl und Cyclohexyl;
   (c) C₃-C₆-Alkenyl, bevorzugt C₃-C₄-Alkenyl wie 2-Propenyl und 2-Butenyl,
   (d) C₅-C₆-Cycloalkenyl wie 2-Cyclopentenyl und 2-Cyclohexenyl,
   (e) C₃-C₆-Alkinyl, bevorzugt C₃-C₄-Alkinyl wie 2-Propinyl, 2-Butinyl und 3-Butinyl, wobei die Alkinylgruppe einen der folgenden Reste tragen kann: Halogen wie Fluor, Chlor, Brom und Jod, C₁-C₄-Alkoxy wie Methoxy und tert.-Butoxy oder Phenyl, welches seinerseits eine bis drei der folgenden Gruppen tragen kann: Halogen, Fluor, Chlor oder Brom, Nitro, Cyano, C₁-C₄-Alkyl wie Methyl, Ethyl oder tert.-Butyl, C₁-C₄-Halogenalkyl wie Fluormethyl, Trifluormethyl, Chlordifluormethly, Pentafluormethyl und 2-Chlor-1,1,2-trifluormethyl oder C₁-C₄-Alkoxy wie Methoxy, Isopropoxy und tert.-Butoxy;
   (f) Phenyl, das eine bis drei der folgenden Gruppen tragen kann:
      - Halogen wie Fluor, Chlor, Brom und Jod, insbesondere Fluor und Chlor, Nitro, Cyano, C₁-C₄-Alkyl wie Methyl, Ethyl und tert.-Butyl, partiell oder vollständig halogeniertes C₁-C₄-Alkyl wie Trifluormethyl, 1,1,2,2-Tetrafluorethyl und Trichlormethyl, C₁-C₄-Alkoxy wie Methoxy, Ethoxy und Isopropoxy oder partiell oder vollständig halogeniertes C₁-C₄-Alkoxy wie Trifluormethoxy, Chloridfluormethoxy, 1-Fluorethoxy, Pentafluorethoxy und 2-Chlor-1,1,2-trifluorethoxy
   (g) N-Phthalimido, Tetrahydrophthalimido, N-Succinimido oder Maleinimido;
R²
   Wasserstoff oder C₁-C₆-Alkyl wie vorstehend genannt, insbesondere Wasserstoff;
R³
   (a) verzweigtes oder unverzweigtes C₁-C₆-Alkyl, bevorzugt C₁-C₄-Alkyl wie Methly, Ethyl, Isopropyl und tert.-Butyl, welches ein bis drei der folgenden Reste tragen kann: Halogen wie Fluor, Chlor und Brom, Cyano, C₁-C₄-Alkoxy wie Methoxy und tert.-Butoxy, C₃-C₈-Cycloalkyl, insbesondere C₃-C₈-Cycloalkyl wie Cyclopropyl, Cyclopentyl und Cyclohexyl oder Phenyl, wobei der Phenylrest seinerseits bis zu drei der folgenden Gruppen tragen kann: Halogen und Fluor, Chlor und Brom, C₁-C₄-Alkyl wie Methyl und tert.-Butyl, C₁-C₄-Halogenalkyl wie Fluormethyl, Trichlormethyl, 2-Chlor- 1,1,2-trifluorethyl und Pentafluorethyl, C₁-C₄-Alkoxy wie Methoxy und tert.-Butoxy, partiell oder vollständig halogeniertes C₁-C₄-Alkoxy wie Fluormethyl, Trichlormethoxy, 2-Chlor-1,1,2-trifluorethoxy und Pentafluorethoxy,
   (b) C₃-C₈-Cycloalkyl, bevorzugt C₃-C₆-Cycloalkyl, insbesondere Cyclopropyl, Cyclopentyl und Cyclohexyl, das jeweils einen bis drei der folgenden Reste tragen kann: Halogen wie Fluor, Chlor und Brom, C₁-C₆-Alkyl, bevorzugt C₁-C₄-Alkyl wie Methyl und tert.-Butyl, partiell oder vollständig halogeniertes C₁-C₆-Alkyl, bevorzugt C₁-C₄-Halogenalkyl wie Fluormethyl, Trichlormethyl, 2-Chlor-1,1,2-trifluorethyl und Pentafluorethyl, C₁-C₄-Alkoxy wie Methoxy und tert.-Butoxy, oder partiell oder vollständig halogeniertes C₁-C₄-Alkoxy wie Fluormethoxy, Trichlormethoxy, 2-Chlor-1,1,2-trifluorethoxy und Pentafluorethoxy;
   (c) verzweigtes oder unverzweigtes C₃-C₆-Alkenyl oder verzweigtes oder unverzweigtes C₃-C₆-Alkinyl, bevorzugt C₃-C₄-Alkenyl oder C₃-C₄-Alkinyl wie 2-Propenyl, 2-Butenyl, 2-Propinyl, 1,1-Dimethyl-2-propinyl und 3-Butinyl, welches jeweils bis zu dreifach durch Halogen wie Fluor, Chlor oder Brom und/oder einmal durch Phenyl substituiert sein kann, wobei der Phenylrest seinerseits eine bis drei der folgenden Substituenten tragen kann: Halogen, insbesondere Fluor und Chlor, Cyano, Nitro, C₁-C₄-Alkyl wie Methyl und tert.-Butyl, partiell oder vollständig halogeniertes C₁-C₄-Alkyl wie Fluormethyl, Trifluormethyl, Trichlormethyl, 2-Chlor-1,1,2-trifluorethyl und Pentafluorethyl, C₁-C₄-Alkoxy wie Methoxy und tert.-Butoxy, C₁-C₄-Halogenalkoxy wie Fluormethoxy, Trifluormethoxy, Trichlormethoxy, 2-Chlor-1,1,2-trifluorethoxy und Pentafluorethoxy, C₁-C₄-Alkylthio wie Methylthio und tert.-Butylthio, partiell oder vollständig halogeniertes C₁-C₄-Alkylthio wie Fluormethylthio, Trifluormethylthio, Trichlormethylthio, 2-Chlor-1,1,2-trifluorethylthio und Pentafluorethylthio;
   (d) Phenyl, welches eine bis vier der folgenden Gruppen tragen kann: C₁-C₄-Alkyl wir vorstehend genannt, insbesondere Methyl, Ethyl und 1-Methylethyl; partiell oder vollständig halogeniertes C₁-C₄-Alkyl wie vorstehend genannt, insbesondere Trifluormethyl und Chloridfluormethyl; C₁-C₄-Alkoxy wie vorstehend genannt, insbesondere Methoxy und Ethoxy; partiell oder vollständig halogeniertes C₁-C₄-Alkoxy wie vorstehend genannt, insbesondere Trifluormethoxy, Trichlormethoxy und Pentafluorethoxy; C₁-C₄-Alkylthio wie vorstehend genannt, insbesondere Dilfuormethylthio, Trifluormethylthio und Pentafluormethylthio, Halogen wie vorstehend genannt, insbesondere Fluor und Chlor, Cyano, Nitro, Formyl, C₁-C₄-Alkanoyl wie Acetyl, Propionyl, Butyryl, insbesondere Acetyl, partiell oder vollständig halogeniertes Alkanoyl wie Trifluoracetyl, Trichloracetyl, Pentafluorpropionyl, insbesondere Trifluoracetyl oder Alkoxycarbonyl wie Methoxycarbonyl und tert.-Butoxycarbonyl;
sowie die pflanzenverträglichen Salze derjenigen Verbindungen, in denen R² für Wasserstoff steht.

Als Salze der Verbindungen der Formel I kommen landwirtschaftlich brauchbare Salze, beispielweise Alkalimetallsalze, insbesondere Natrium- und Kaliumsalze, Erdalkalimetallsalze wie insbesondere Calzium-, Magnesium- und Bariumsalze, Mangan-, Kupfer-, Zink- oder Eisensalze sowie Ammoniumsalze wie Tetraalkyl- und Benzyltrialkylammoniumsalze, Phosphonium-, Sulfoniumsalze wie Trialkylsulfoniumsalze oder Sulfoxoniumsalze in Betracht.

Die Verbindungen I bzw. die sie enthaltenden herbiziden Mittel sowie deren umweltverträgliche Salze von Alkalimetallen und Erdalkalimetallen können in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin. alkylierte Naphthaline oder deren Derivate, Methanol, Ethanol, Propanol, Butanol, Cyclohexanol, Cyclohexanon oder stark polare Lösungsmittel, N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrace als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz- Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldeyhd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Tragerstoffe sind Mineralerden wie Silicagel, Kieselsäuren, Kieselgele, Silikate, Talkum, Kaolin, Kalkstein, Kalk. Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe und pflanzliche Produkte wie Getreidemehl, Baumrinden, Holz- und Nußschalenmehl, Cellulosepulver oder andere feste Trägerstoffe.

Die Formulierungen enthalten im allgemeinen zwischen 0,01 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
- I: 20 Gew.-Teile der Verbindung Nr. 1.001 werden in einer Mischung gelöst, die aus 80 Gew.-Teilen alkyliertem Benzol, 10 Gew.-Teilen des Anlagerungsproduktes von 8 bis 10 mol Ethylenoxid an 1 mol Ölsäure-N-monoethanolamid, 5 Gew.-Teilen Calciumsalz der Dodecylbenzolsulfonsäure und 5 Gew.-Teilen des Anlagerungsproduktes von 40 mol Ethylenoxid an 1 mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.
- II: 20 Gew.-Teile der Verbindung Nr. 1.001 werden in einer Mischung gelöst, die aus 40 Gew.-Teilen Cyclohexanon, 30 Gew.-Teilen Isobutanol, 20 Gew.-Teilen des Anlagerungsproduktes von 7 mol Ethylenoxid an 1 mol Isooctylphenol 1 und 10 Gew.-Teilen des Anlagerungsproduktes 40 mol Ethylenoxid an 1 mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
- III: 20 Gew.-Teile des Wirkstoffs Nr. 1.001 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusol besteht. Durch Eingießen und feines Verteilen der Lösung in 100 000 Gew.-Teilen Wasser erhält man eine wäßrige Dispersion, die 0,02 Gew.-% des Wirkstoffs enthält.
- IV: 20 Gew.-Teile des Wirkstoffs Nr. 1.001 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.
- V: 3 Gew.-Teile des Wirkstoffs Nr. 1.001 werden mit 97 Gew.-Teilen feinteiligem Kaolin vermischt. Man erhält auf diese Weise ein Stäubemittel, das 3 Gew.-% des Wirkstoffs enthält.
- VI: 20 Gew.-Teile des Wirkstoffs Nr. 1.001 werden mit 97 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teile eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 2,0 kg/ha aktive Substanz (a.S.).

In Anbetracht der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor-Farberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Isoxazolcarbonsäureamide der allgemeinen Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineral salzlösungen, welche zur Behebung von Ernährungs- und Spurenelementmängeln eingesetzt werden. Es kannen auch nichtphytotoxische Öle und Ölkonzentrate zugesetzt werden.

### Synthesebeispiele

### Herstellung von Hydroxamsäurechloriden III:

Cyclopropylcarbamoylformhydroxamoylchlorid 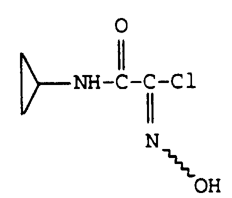 28,5 g (0,5 mol) Cyclopropylamin werden in 500 ml Wasser bei Raumtemperatur vorgelegt und anschließend 42,0 g (0,5 mol) Diketen zugetropft. Dabei sinkt der pH-Wert von 12.0 auf 5.5-6.5 ab. Man rührt 10 min nach, versetzt mit 37,9 g (0,55 mol) Natriumnitrit und gibt anschließend ca. 75 ml konz. Salzsäure so zu, daß der pH-Wert stets oberhalb von 4.5 bleibt. Nach vollendeter Zugabe werden bei Raumtemperatur 41,1 g (0,58 mol) elementares Chlor eingegast. Der vollständige Umsatz wird mittels DC oder HPLC kontrolliert. Das bei der Reaktion entstehende Hydroxamsäurechlorid kann entweder durch Filtration bei 0°C oder durch mehrfache Extraktion mit Ethylacetat und Abziehen des Solvens im Vakuum isoliert werden. Der so erhaltene Feststoff wird mit Wasser gewaschen und bei maximal 40°C im Vakuum getrocknet. Man erhält 72,1 g (89 %) Cyclopropylcarbamoylformhydroxamoylchlorid als weißen Feststoff.
¹H-NMR (250 MHz, DMSO): δ = 0,50-0,78 (m; 4H), 2,75 (m; 1H), 8,48 (d; 1H, NH), 12,80 (s; 1H, OH)

### Herstellung der Isoxazolcarbonsäureamide I:

3-tert.-Butylaminocarbonyl-isoxazol-4-carbonsäuremethylester (Beispiel 1.001) 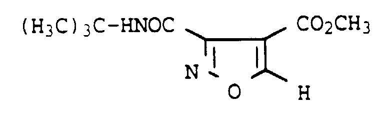 15,5 g (0,1 mol) 3-(1-Pyrrolidinyl)-acrylsäuremethylester werden in 200 ml THF gelöst und mit 17,8 g (0,1 mol) tert.-Butylcarbamoylformhydroxamoylchlorid in 200 ml THF versetzt. Man erhitzt auf Rückflußtemperatur für ca. 3 Stunden. Anschließend wird mit Wasser verdünnt, mit Natronlauge alkalisch gestellt, 5 Minuten gerührt und dann mit Salzsäure wieder angesäuert. Die Lösung wird mit Essigsäureethylester extrahiert und die organische Phase mit 2 N Salzsäure und. mit Wasser gewaschen. Die Reinigung erfolgt durch Chromatographie an Kieselgel. Ausbeute 12,9 g. Fp. 91-93°C.

3-tert.-Butylaminocarbonyl-isoxazol-4-carbonsäuremethylester (Beispiel 1.001) 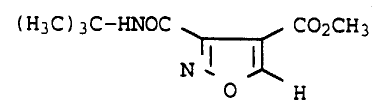 64,5 g (0,5 mol) 3-Dimethylamino-acrylsäuremethylester werden in 200 ml THF gelöst und mit 90,0 g (0,5 mol) tert.-Butylcarbamoylformhydroxamoylchlorid in 200 ml THF versetzt. Man erhitzt auf Rückflußtemperatur für ca. 3 Stunden. Anschließend wird mit Wasser verdünnt, mit Natronlauge neutral gestellt, 5 Minuten gerührt und dann mit Salzsäure wieder angesäuert. Die Lösung wird mit Essigsäureethylester extrahiert und die organische Phase mit 2 N Salzsäure und mit Wasser gewaschen, getrocknet und eingeengt. Ausbeute 110 g. Fp. 91-93°C.

3-tert.-Butylaminocarbonyl-isoxazol-4-carbonsäure 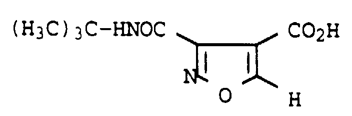 7,2 g (0,032 mol) 3-tert.-Butylaminocarbonyl-isoxazol-4-carbonsäuremethylester werden in 80 ml Methanol gelöst und bei 0°C mit 1,34 g (0,033 mol) Natriumhydroxid in 50 ml Wasser versetzt. Nach 12-stündigem Rühren wird mit Wasser verdünnt, mit Salzsäure auf pH 1 gestellt und dreimal mit Essigsäureethylester extrahiert. Die organische Phase wird über Magnesiumsulfat getrocknet und eingeengt. Ausbeute 5,7 g eines Öls, welches ohne weitere Reinigung in den Folgeumsetzungen eingesetzt werden kann.

3-tert.-Butylaminocarbonyl-isoxazol-4-carbonsäurepropargylester (Beispiel 1.010) 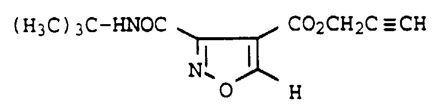 4,3 g (0,02 mol) 3-tert.-Butylaminocarbonyl-isoxazol-4-carbonsäure und 1,5 g (0,0265 mol) Propargylalkohol werden in 150 ml Essigsäureethylester gelöst und mit 7,6 g (0,075 mol) N-Methylmorpholin und 2,5 g (0,02 mol) N,N-Dimethylaminopyridin versetzt. Man läßt fünf Minuten bei Raumtemperatur rühren, tropft dann 17,8 g einer 50 %igen Propanphosphonsäureanhydrid-Lösung in Dichlormethan zu und läßt dann acht Stunden bei 45°C und weitere 14 Stunden bei Raumtemperatur rühren. Zur Aufarbeitung wird die Lösung mit gesättigter wäßriger Natriumhydrogencarbonat- und Zitronensäurelösung extrahiert, über Magnesiumsulfat getrocknet, eingeengt und an Kieselgel chromatographiert. Ausbeute 3 g.

Isoxazol-3,4-dicarbonsäure-3-tert.-butylester-4-ethylester 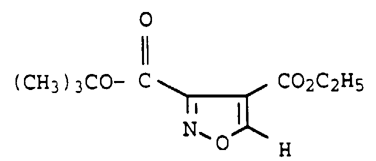 87,7 g (0,52 mol) 3-(1-Pyrrolidinyl)-acrylsäureethylester werden in 500 ml THF gelöst und mit 93,2 g (0,52 mol) 2-Chlor-2-hydroximinoessigsäure-tert.-butylester in 500 ml THF versetzt. Man erhitzt auf Rückflußtemperatur für ca. 3 Stunden. Anschließend wird mit Wasser verdünnt, mit Natronlauge neutral gestellt, 5 Minuten gerührt und dann mit Salzsäure wieder angesäuert. Die Lösung wird mit Essigsäureethylester extrahiert und die organische Phase mit 2 N Salzsäure und mit Wasser gewaschen. Die Reinigung erfolgt durch Chromatographie an Kieselgel. Ausbeute 50,6 g eines Öls.

Isoxazol-3,4-dicarbonsäure-4-ethylester 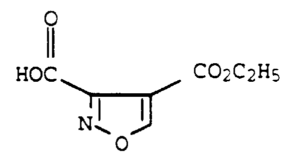 30 g (0,125 mol) Isoxazol-3,4-dicarbonsäure-3-tert.butylester-4-ethylester werden in 600 ml Dichlormethan und 60 ml Trifluoressigsäure zwei Stunden bei Raumtemperatur gerührt. Die Reaktionslösung wird auf 1 1 Wasser gegossen, die organische Phase wird abgetrennt, mehrfach mit Wasser extrahiert, getrocknet und eingeengt. Man erhält 21 g eines Öls, welches ohne weitere Reinigung eingesetzt wird.

Isoxazol-4-carbonsäureethylester-3-carbonsäurechlorid 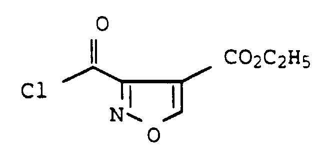 16,9 g (0,09 mol) Isoxazol-3,4-dicarbonsäure-4-ethylester werden in 200 ml Toluol gelöst, mit 21,7 g (0,18 mol) Thionylchlorid versetzt und 2 h zur Rückflußtemperatur erhitzt. Anschließend engt man zur Trockene ein und setzt das erhaltene Öl (17,4 g) ohne weitere Reinigung weiter um.

3-(1,1-Dimethylpropargyl)aminocarbonyl-isoxazol-4-carbonsäureethylester (Beispiel 1.027) 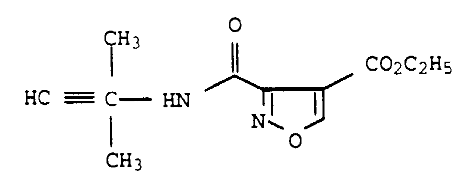 5,8 g (0.028 mol) Isoxazol-4-carbonsäureethylester-3-carbonsäurechlorid werden in 100 ml Toluol gelöst, die Lösung wird auf 0-10°C gekühlt und mit 4.73 g (o.057 mol) 1,1-Dimethylpropargylamin versetzt. Man läßt einige Stunden bei Raumtemperatur rühren, versetzt die Lösung dann mit Wasser, trennt die organische Phase ab und extrahiert diese mit waßriger Citronensäure und waßriger Natriumhydrogencarbonatlösung. Anschließend engt man zur Trockene ein und reinigt den erhaltenen Rückstand durch Chromatrographie an Kieselgel. Ausbeute 3.5 g, Fp. 75-82°C

Entsprechend den vorstehenden Beispielen wurden die in Tabelle I aufgeführten Verbindungen hergestellt.

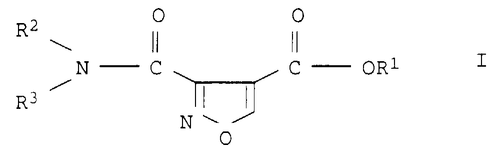

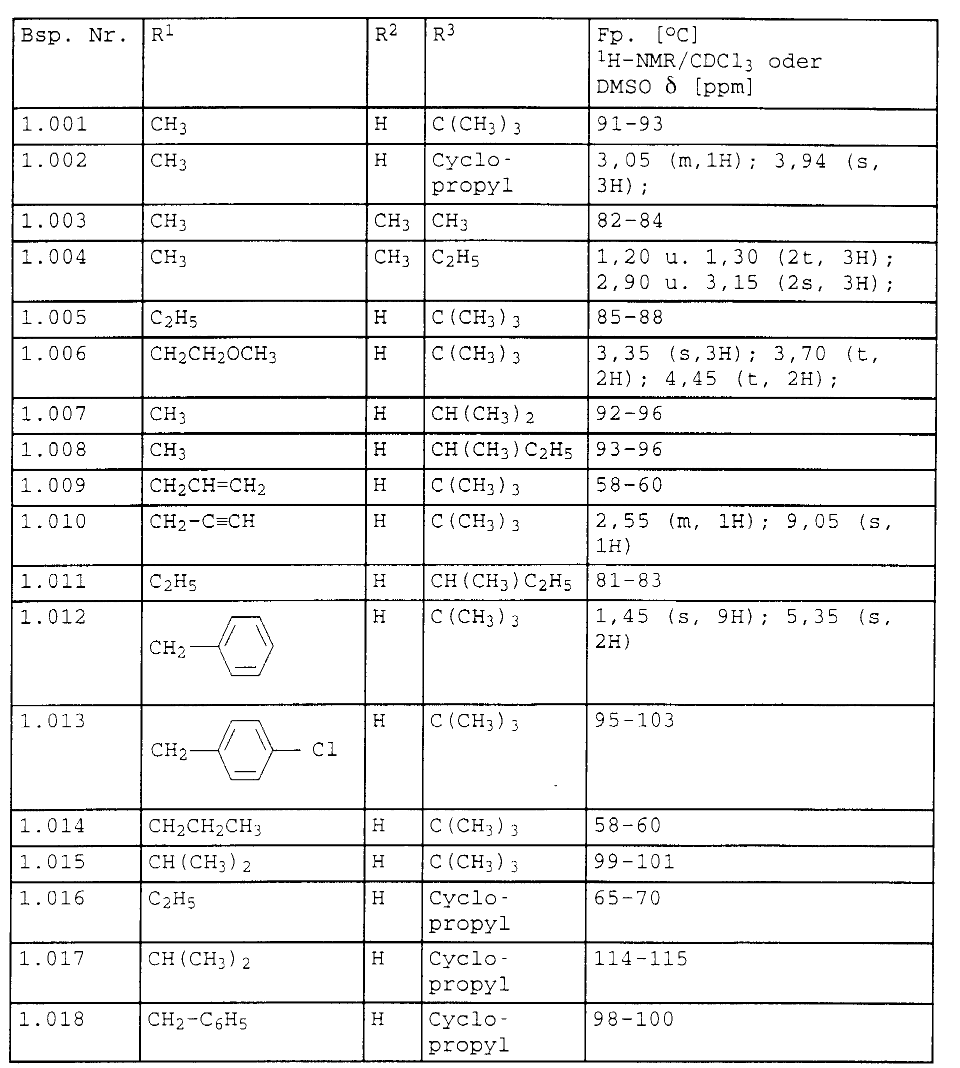

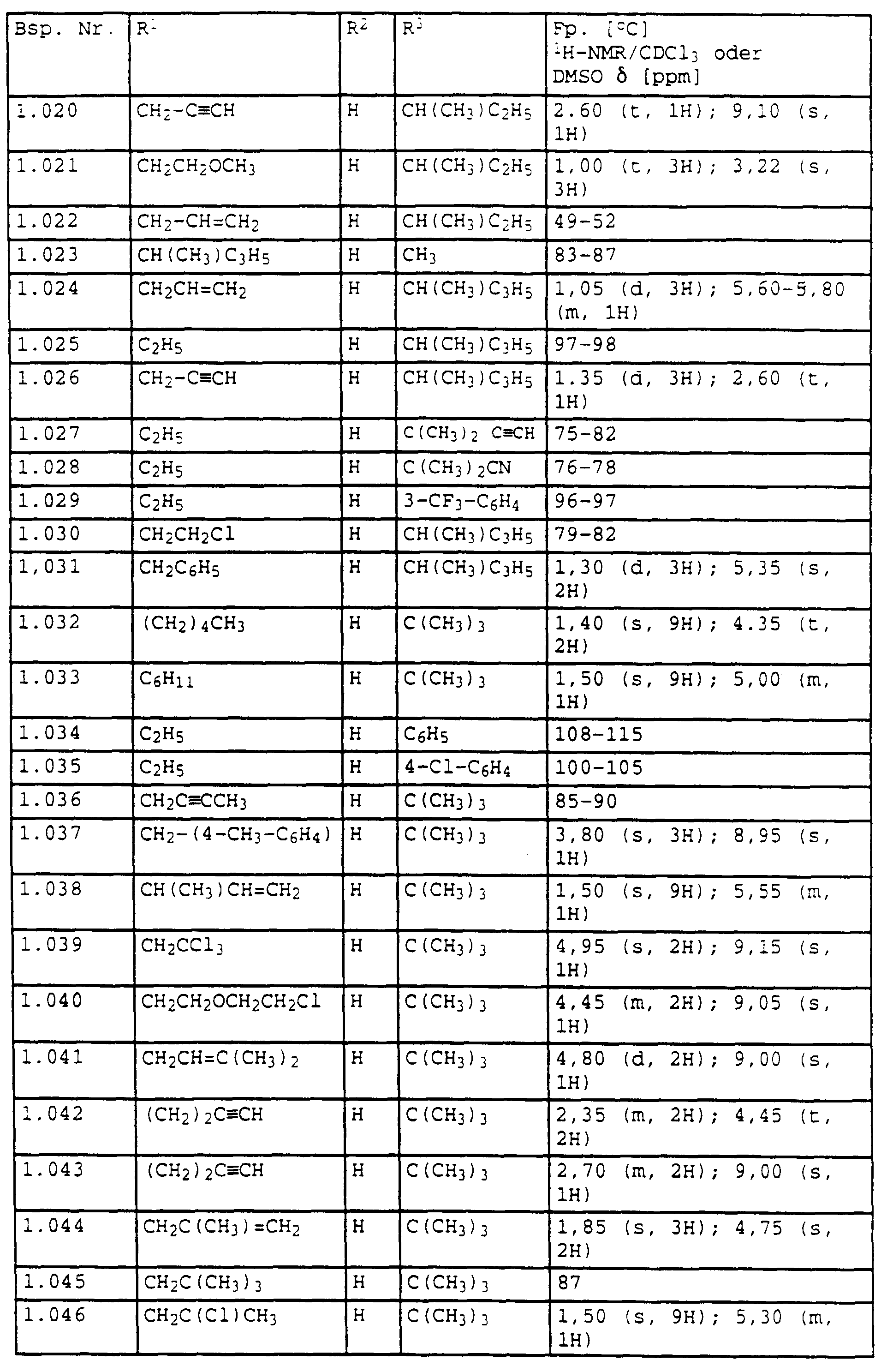

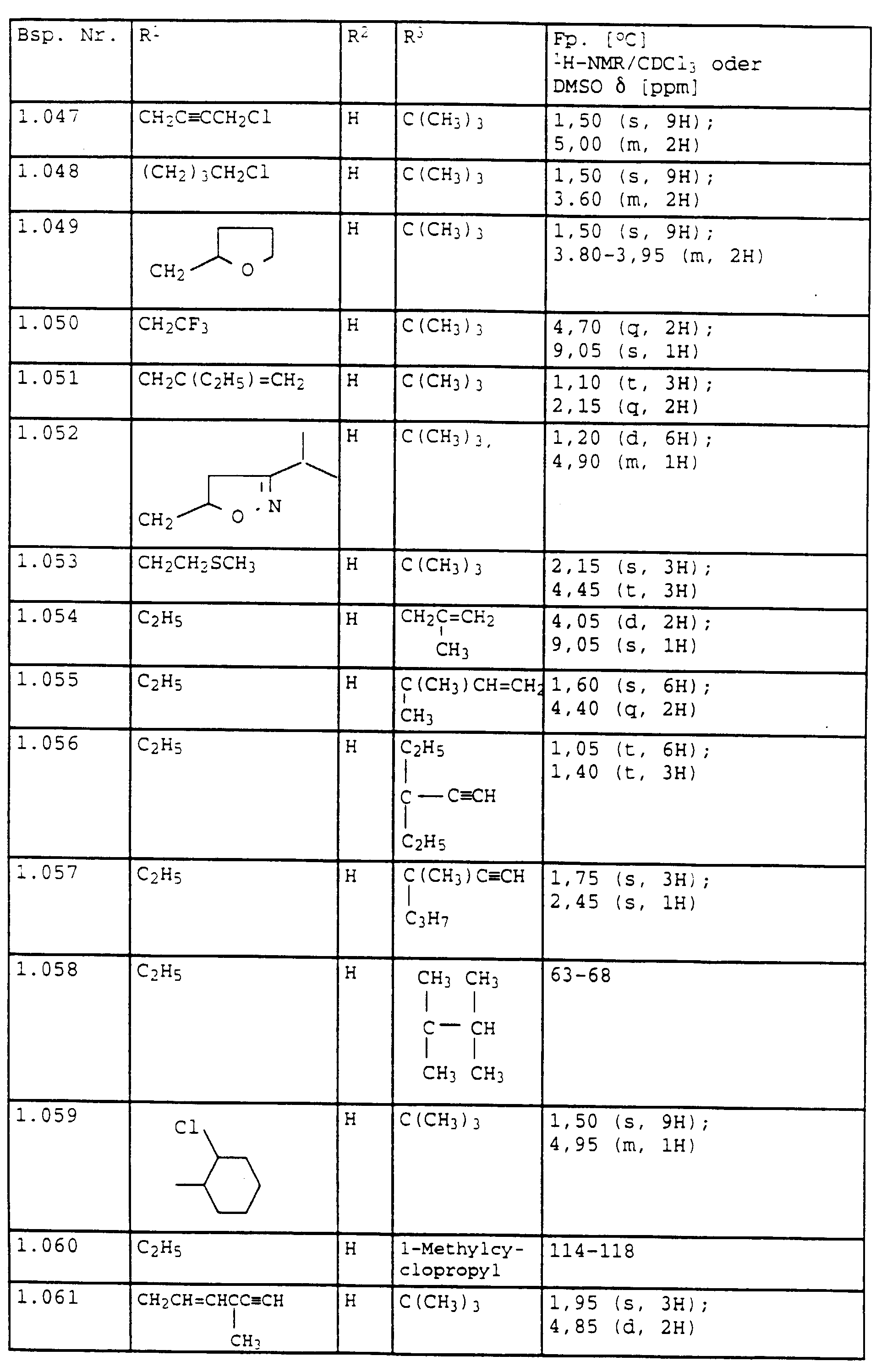

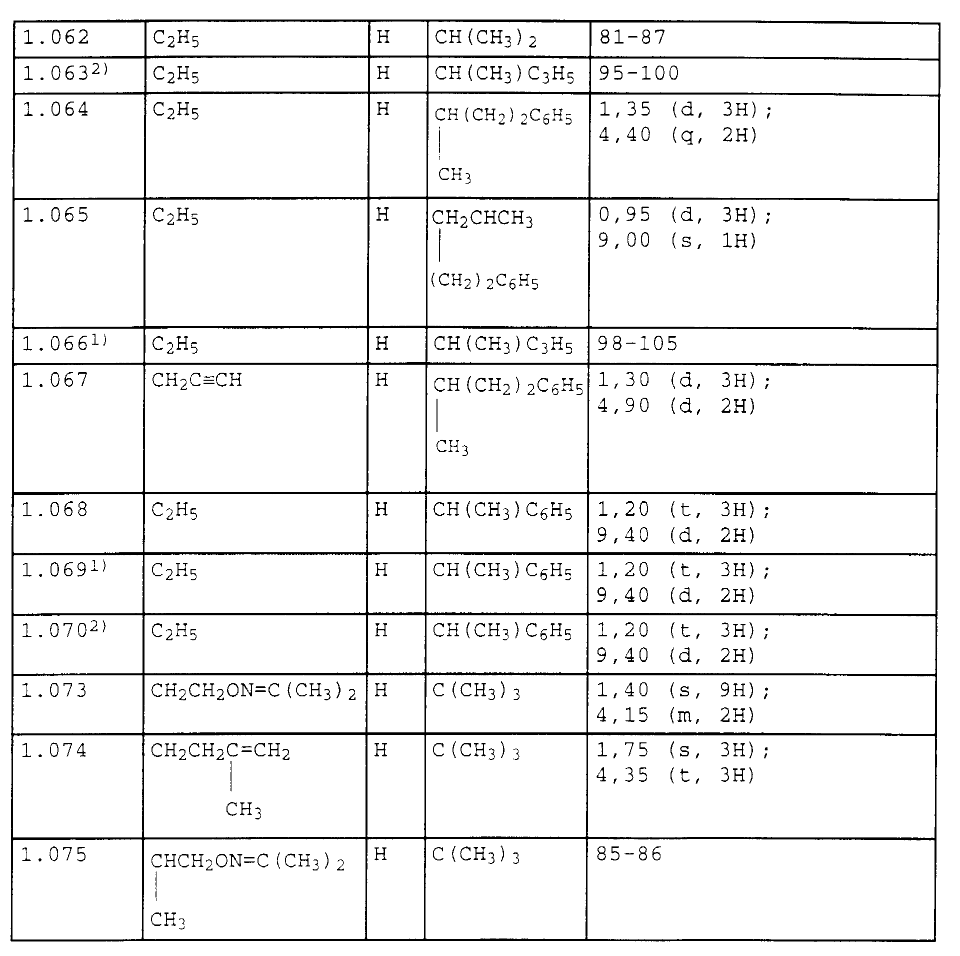

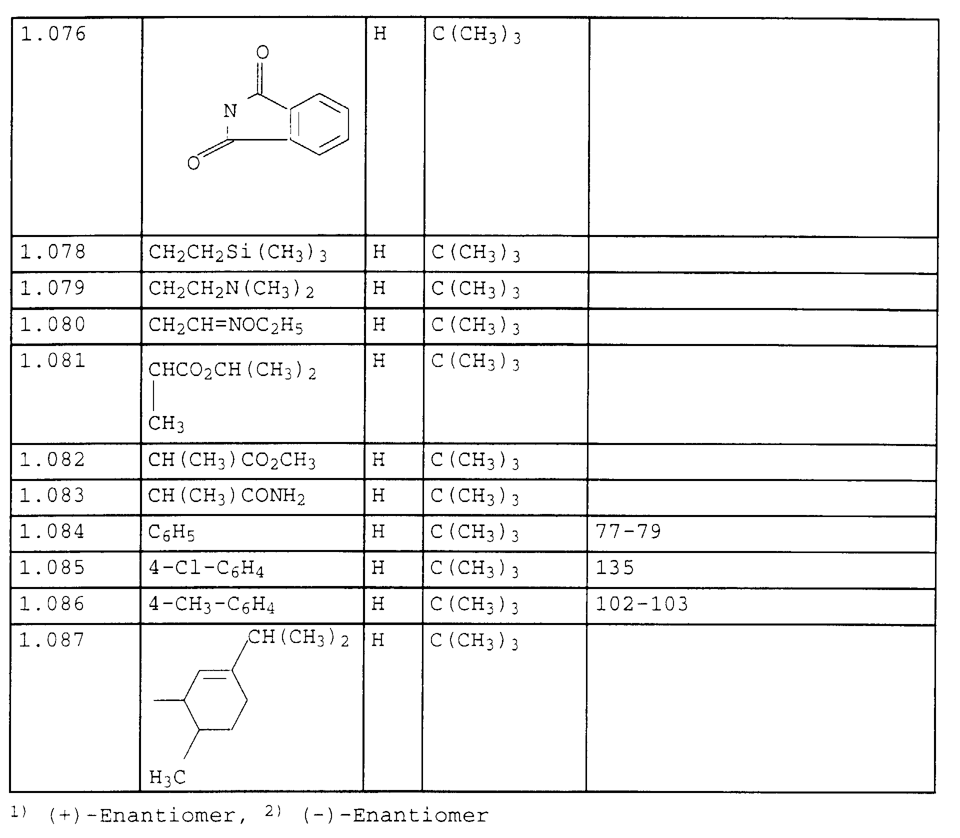

### Anwendungsbeispiele

Die herbizide Wirkung der Isoxazolcarbonsäureamide der allgemeinen Formel I ließ sich durch Gewächshausversuche zeigen:

Als Kulturgefäße dienten Plastiblumentöpfe mit lehmigem Sand mit etwa 3,0 % Humus als Substrat. Die Samen der Testpflanzen wurden nach Arten getrennt eingesetzt.

Zum Zweck der Nachauflaufbehandlung werden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung beträgt 3,0 kg/ha a.S.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Abkürzung |
|---|---|---|
| Centaurea cyanus | Kornblume | CENCY |
| Galium aparine | Klettenlabkraut | GALAP |
| Viola ssp. | Stiefmütterchen | VIOSS |

Die nachfolgende Gegenüberstellung in Tabelle 1 demonstriert die überlegene herbizide Wirkung der erfindungsgemäßen Verbindung Nr. 1.001 im Vergleich zu der aus EP-A 418 667, Beispiel Nr. 3.116 bekannten Vergleichsverbindung A.

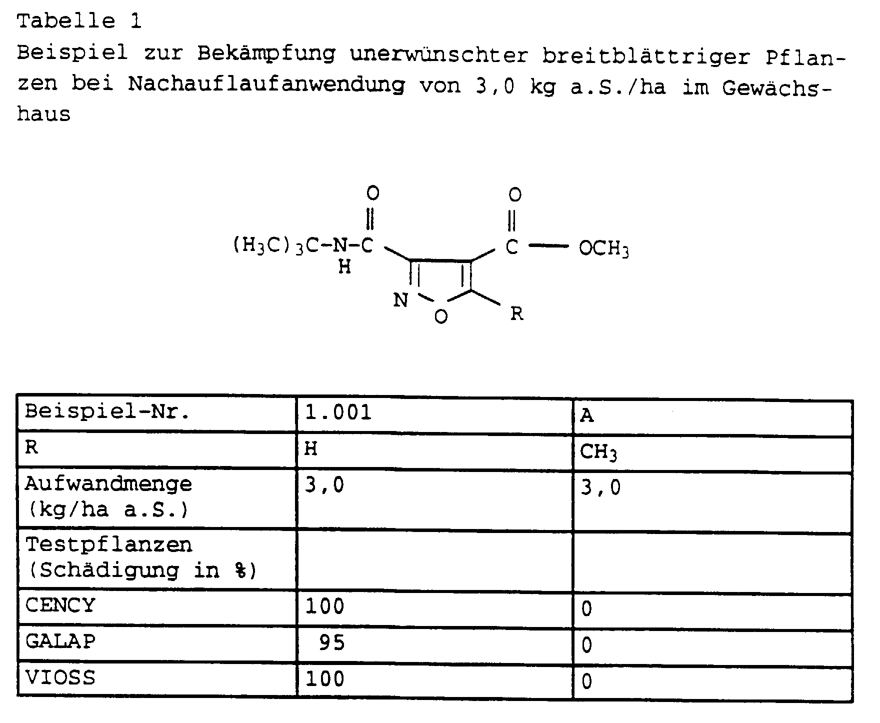

## Patentansprüche

1. Isoxazolcarbonsäureamide der Formel I 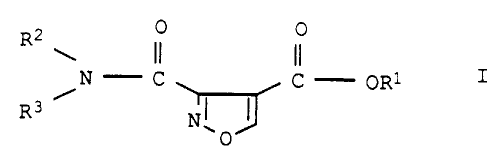 in der die Substituenten die folgende Bedeutung haben:
R¹
(a) eine C₁-C₆-Alkylgruppe, welche ein bis fünf Halogenatome und/ oder bis zu drei Hydroxy- und/oder C₁-C₄-Alkoxygruppen und/ oder einen der folgenden Reste tragen kann:
- Cyano;
- C₁-C₄-Alkoxy-C₂-C₄-alkoxy;
- C₁-C₃-Alkylthio;
- C₁-C₃-Alkylamino, Di-(C₁-C₃-alkyl)amino;
- C₃-C₆-Cycloalkylamino oder Di-(C₃-C₈-cycloalkyl)amino;
- Tri(C₁-C₄-alkyl)silyl;
- Carboxyl, C₁-C₃-Alkoxycarbonyl oder C₁-C₃-Alkoxycarbonyl-C₁-C₃-alkoxy, Di(C₁-C₃-alkyl)aminocarbonyl
- C₁-C₆-Alkaniminoxy oder C₅-C₆-Cycloalkaniminoxy;
- einen 5- oder 6-gliedrigen gesättigten oder ungesättigten heterocyclischen Rest mit jeweils bis zu 3 Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, C₁-C₃-Alkyl oder C₁-C₃-Alkoxy;
- C₃-C₆-Cycloalkyl;
- Phenyl, das noch bis zu drei der folgenden Substituenten tragen kann: Halogen, Nitro, Cyano, C₁-C₃-Alkyl, partiell oder vollständig halogeniertes C₁-C₃-Alkyl, C₁-C₃-Alkoxy oder partiell oder vollständig halogeniertes C₁-C₃-Alkoxy;
- einen Rest -CR¹⁰=N-R¹¹, wobei R¹⁰ und R¹¹ die folgende Bedeutung haben:
R¹⁰ Wasserstoff oder C₁-C₆-Alkyl und
R¹¹ C₁-C₆-Alkoxy, C₃-C₆-Alkenyloxy oder C₃-C₆-Alkinyloxy, die jeweils bis zu drei Halogenatome und/oder einen Phenylrest mit gewünschtenfalls bis zu drei der folgenden Reste tragen können: Halogen, Nitro, Cyano, C₁-C₃-Alkyl oder C₁-C₃-Alkoxy;
C₁-C₆-Alkylamino, Di-(C₁-C₆-alkyl)amino oder Phenylamino, wobei der Phenylrest zusätzlich bis zu drei der folgenden Reste tragen kann: Halogen, Nitro, Cyano, C₁-C₃-Alkyl oder C₁-C₃-Alkoxy;
(b) C₃-C₈-Cycloalkyl, das einen bis fünf der folgenden Reste tragen kann: Halogen, C₁-C₄-Alkyl, C₁-C₄-Alkoxy partiell oder vollständig halogeniertes C₁-C₄-Alkyl oder partiell oder vollständig halogeniertes C₁-C₄-Alkoxy;
(c) gegebenenfalls durch C₃-C₆-Alkinyl, Halogen, C₁-C₄-Alkoxy oder Phenyl substituiertes verzweigtes oder unverzweigtes C₃-C₆-Alkenyl,
(d) C₅-C₆-Cycloalkenyl,
(e) gegebenenfalls durch Halogen, C₁-C₄-Alkoxy oder Phenyl substituiertes verzweiges oder unverzweigtes C₃-C₆-Alkinyl, wobei der Phenylrest seinerseits eine bis drei der folgenden Gruppen tragen kann: Halogen, Nitro, Cyano, C₁-C₄-Alkyl, partiell oder vollständig halogeniertes C₁-C₄-Alkyl oder C₁-C₄-Alkoxy, partiell oder vollständig halogeniertes C₁-C₄-Alkoxy;
(f) Phenyl, das eine bis drei der folgenden Gruppen tragen kann:
- Halogen, Nitro, Cyano, C₁-C₄-Alkyl, partiell oder vollständig halogeniertes C₁-C₄-Alkyl, C₁-C₄-Alkoxy oder partiell oder vollständig halogeniertes C₁-C₄-Alkoxy;
(g) N-Phthalimido, Tetrahydrophthalimido, N-Succinimido oder Maleinimido;
R²
Wasserstoff oder C₁-C₆-Alkyl;
R³
(a) eine C₁-C₆-Alkylgruppe, die einen bis drei der folgenden Reste tragen kann: Halogen, Cyano, C₁-C₄-Alkoxy, C₃-C₈-Cycloalkyl oder Phenyl, wobei der Phenylring seinerseits einen bis drei der folgenden Reste tragen kann: Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy,
(b) eine C₃-C₈-Cycloalkylgruppe, die einen bis drei der folgenden Reste tragen kann: Halogen, C₁-C₆-Alkyl, partiell oder vollständig halogeniertes C₁-C₆-Alkyl, C₁-C₄-Alkoxy oder partiell oder vollständig halogeniertes C₁-C₄-Alkoxy;
(c) eine verzweigte oder unverzweigte C₃-C₈-Alkenyl- oder verzweigte oder unverzweigte C₃-C₈-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro;
(d) eine Phenylgruppe, die eine bis vier der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Nitro, Cyano, Formyl, C₁-C₄-Alkanoyl, C₁-C₄-Halogenalkanoyl oder C₁-C₄-Alkoxycarbonyl;
sowie die pflanzenverträglichen Salze derjenigen Verbindungen, in denen R² für Wasserstoff steht.

2. Isoxazolcarbonsäureamide der Formel I gemäß Anspruch 1, in der R¹ für C₁-C₆-Alkyl oder C₅-C₆-Cycloalkyl steht.

3. Isoxazolcarbonsäureamide der Formel I gemäß Anspruch 1, in der R² für Wasserstoff steht.

4. Isoxazolcarbonsäureamide der Formel I gemäß Anspruch 1, in der R³ für C₁-C₆-Alkyl steht.

5. Isoxazolcarbonsäureamide der Formel I gemäß Anspruch 1, in der R¹ für C₁-C₆-Alkyl oder C₅-C₆-Cycloalkyl, R³ für C₁-C₆-Alkyl und R² für Wasserstoff stehen.

6. Verfahren zur Herstellung von Isoxazolcarbonsäureamiden der Formel I', 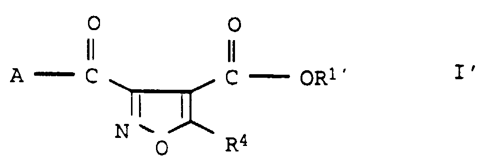 in der die Substituenten die folgende Bedeutung haben:
R^{1'} ggf. substituiertes Alkyl, Cycloalkyl, Phenyl, Alkenyl oder Alkinyl;
A eine Gruppe NR^{2'}R^{3'} oder eine Gruppe OR⁵;
R^{2'} Wasserstoff oder Alkyl;
R^{3'} ggf. substituiertes Alkyl, Cycloalkyl, Alkenyl, Alkinyl oder Aryl;
oder R^{2'} und R^{3'} gemeinsam -CH₂CH₂CH₂CH₂- oder -CH₂CH₂CH₂CH₂CH₂-, wobei in diesen Ketten eine CH₂-Gruppe durch Sauerstoff, Schwefel oder NCH₃ ersetzt sein kann;
R⁵ ggf. substituiertes Alkyl, Cycloalkyl,Phenyl, Alkenyl oder Alkinyl;
R⁴ Wasserstoff oder Alkyl,
dadurch gekennzeichnet, daß man ein Enamin der Formel II 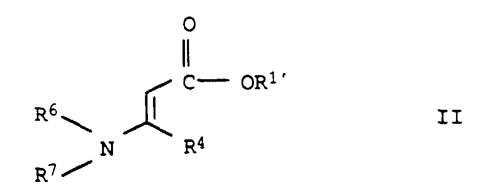 in der R⁶ und R⁷ für Wasserstoff oder C₁-C₄-Alkyl stehen oder gemeinsam -CH₂CH₂CH₂CH₂- oder -CH₂CH₂CH₂CH₂CH₂- bedeuten, wobei in diesen Ketten eine CH₂-Gruppe durch Sauerstoff, Schwefel oder NCH₃ ersetzt sein kann, mit einem Hydroxamsäurechlorid der Formel III 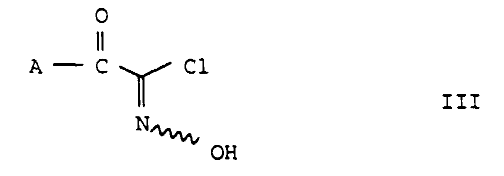 umsetzt.

7. Verfahren zur Herstellung der Isoxazolcarbonsäureamide der Formel I gemäß Anspruch 1, dadurch gekennzeichnet, daß man ein Isoxazol-3-carbonsäureamid-4-alkylester in an sich bekannter Weise in Gegenwart einer Base zur Säure IV 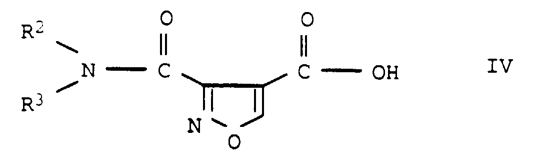 spaltet und die so erhaltene Säure in an sich bekannter Weise mit einem Alkohol der Formel V
HOR¹ V
zur Verbindung I verestert.

8. Herbizide Mittel enthaltend ein Isoxazolcarbonsäureamid der Formel I gemäß Anspruch 1 sowie inerte Zusatzstoffe.

9. Verfahren zur Bekämpfung unerwünschten Pflanzenwuchses, dadurch gekennzeichnet, daß man die Pflanzen und/oder ihren Lebensraum mit einer herbizid wirksamen Menge eines Isoxazolcarbonsäureamids der Formel I gemäß Anspruch 1 behandelt.

10. Verwendung der Isoxazolcarbonsäureamide der Formel I gemäß Anspruch 1 als Herbizide.

## Claims

1. An isoxazolecarboxamide of the formula I 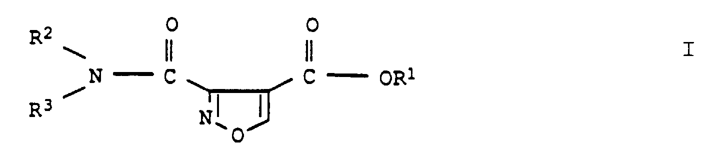 where
R¹ is
(a) C₁-C₆-alkyl which may carry from one to five halogen atoms or up to three hydroxyl or C₁-C₄-alkoxy groups or one of the following radicals:
cyano;
C₁-C₄-alkoxy-C₂-C₄-alkoxy;
C₁-C₃-alkylthio;
C₁-C₃-alkylamino, di-C₁-C₃-alkylamino;
C₃-C₆-cycloalkylamino or di-C₃-C₆-cycloalkylamino; tri-C₁-C₄-alkylsilyl;
carboxyl, C₁-C₃-alkoxycarbonyl or C₁-C₃-alkoxycarbonyl-C₁-C₃-alkoxy, di-(C₁-C₃-alkyl)-aminocarbonyl;
C₁-C₆-alkyliminoxy or C₅- or C₆-cycloalkyliminoxy;
a 5-membered or 6-membered saturated or unsaturated heterocyclic radical, each having up to 3 hetero atoms selected from the group consisting of oxygen, sulfur and nitrogen, where two oxygen or sulfur atoms cannot be directly adjacent and where the heterocycles may furthermore carry one or two of the following substituents: halogen, C₁-C₃-alkyl or C₁-C₃-alkoxy;
C₃-C₆-cycloalkyl;
phenyl which may furthermore carry up to three of the following substituents: halogen, nitro, cyano, C₁-C₃-alkyl, partially or completely halogenated C₁-C₃-alkyl, C₁-C₃-alkoxy or partially or completely halogenated C₁-C₃-alkoxy;
-CR¹⁰=N-R¹¹, where
R¹⁰ is hydrogen or C₁-C₆-alkyl and
R¹¹ is C₁-C₆-alkoxy, C₃-C₆-alkenyloxy or C₃-C₆-alkynyloxy, each of which may carry up to three halogen atoms or a phenyl radical having, if desired, up to three of the following radicals: halogen, nitro, cyano, C₁-C₃-alkyl or C₁-C₃-alkoxy;
C₁-C₆-alkylamino, di-C₁-C₆-alkylamino or phenylamino, where the phenyl radical may additionally carry up to three of the following radicals: halogen, nitro, cyano, C₁-C₃-alkyl or C₁-C₃-alkoxy;
(b) C₃-C₈-cycloalkyl which may carry from one to five of the following radicals: halogen, C₁-C₄-alkyl, C₁-C₄-alkoxy, partially or completely halogenated C₁-C₄-alkyl or partially or completely halogenated C₁-C₄-alkoxy;
(c) branched or straight-chain C₃-C₆-alkenyl which is unsubstituted or substituted by C₃-C₆-alkynyl, halogen, C₁-C₄-alkoxy or phenyl,
(d) C₅- or C₆-cycloalkenyl,
(e) branched or straight-chain C₃-C₆-alkynyl which is unsubstituted or substituted by halogen, C₁-C₄-alkoxy or phenyl, where the phenyl radical in turn may carry from one to three of the following groups: halogen, nitro, cyano, C₁-C₄-alkyl, partially or completely halogenated C₁-C₄-alkyl, C₁-C₄-alkoxy or partially or completely halogenated C₁-C₄-alkoxy;
(f) phenyl which may carry from one to three of the following groups:
halogen, nitro, cyano, C₁-C₄-alkyl, partially or completely halogenated C₁-C₄-alkyl, C₁-C₄-alkoxy or partially or completely halogenated C₁-C₄-alkoxy;
(g) N-phthalimido, tetrahydrophthalimido, N-succinimido or maleimido;
R² is
hydrogen or C₁-C₆-alkyl;
R³ is
(a) C₁-C₆-alkyl which may carry from one to three of the following radicals: halogen, cyano, C₁-C₄-alkoxy, C₃-C₈-cycloalkyl or phenyl, where the phenyl ring in turn may carry from one to three of the following radicals: halogen, C₁-C₄-alkyl, partially or completely halogenated C₁-C₄-alkyl, C₁-C₄-alkoxy or partially or completely halogenated C₁-C₄-alkoxy;
(b) C₃-C₈-cycloalkyl which may carry from one to three of the following radicals: halogen, C₁-C₆-alkyl, partially or completely halogenated C₁-C₆-alkyl, C₁-C₄-alkoxy or partially or completely halogenated C₁-C₄-alkoxy;
(c) branched or straight-chain C₃-C₈-alkenyl or branched or straight-chain C₃-C₈-alkynyl, each of which may be monosubstituted to trisubstituted by halogen or monosubstituted by phenyl, where the phenyl radical in turn may carry from one to three of the following groups: C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
(d) phenyl which may carry from one to four of the following groups: C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, nitro, cyano, formyl, C₁-C₄-alkanoyl, C₁-C₄-haloalkanoyl or C₁-C₄-alkoxycarbonyl;
and the plant-tolerated salts of those compounds in which R² is hydrogen.

2. An isoxazolecarboxamide of the formula I as claimed in claim 1, wherein R¹ is C₁-C₆-alkyl or C₅- or C₆-cycloalkyl.

3. An isoxazolecarboxamide of the formula I as claimed in claim 1, wherein R² is hydrogen.

4. An isoxazolecarboxamide of the formula I as claimed in claim 1, wherein R³ is C₁-C₆-alkyl.

5. An isoxazolecarboxamide of the formula I as claimed in claim 1, wherein R¹ is C₁-C₆-alkyl or C₅- or C₆-cycloalkyl, R³ is C₁-C₆-alkyl and R² is hydrogen.

6. A process for the preparation of an isoxazolecarboxamide of the formula I' where 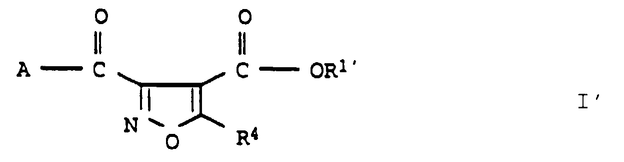
R^{1'} is unsubstituted or substituted alkyl, cycloalkyl, phenyl, alkenyl or alkynyl;
A is NR^{2'}R^{3'} or OR⁵;
R^{2'} is hydrogen or alkyl;
R^{3'} is unsubstituted or substituted alkyl, cycloalkyl, alkenyl, alkynyl or aryl;
or R^{2'} and R^{3'} together are -CH₂CH₂CH₂CH₂- or -CH₂CH₂CH₂CH₂CH₂-, in which chains a CH₂ group may be replaced by oxygen, sulfur or NCH₃;
R⁵ is unsubstituted or substituted alkyl, cycloalkyl, phenyl, alkenyl or alkynyl;
R⁴ is hydrogen or alkyl,
wherein an enamine of the formula II 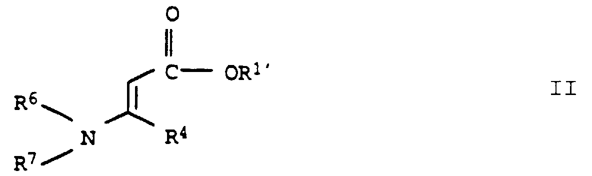 where R⁶ and R⁷ are each hydrogen or C₁-C₄-alkyl or together are -CH₂CH₂CH₂CH₂- or -CH₂CH₂CH₂CH₂CH₂-, in which chains a CH₂ group may be replaced by oxygen, sulfur or NCH₃, is reacted with a hydroxamoyl chloride of the formula III

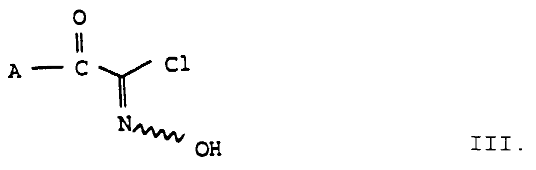

7. A process for the preparation of an isoxazolecarboxamide of the formula I as claimed in claim 1, wherein an alkyl 3-aminocarbonylisoxazole-4-carboxylate is cleaved in a conventional manner in the presence of a base to give an acid IV 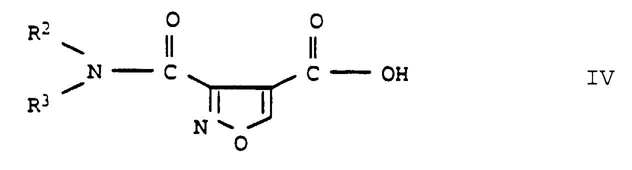 and the acid thus obtained is esterified in a conventional manner with an alcohol of the formula V
HOR¹ V
to give a compound I.

8. A herbicide containing an isoxazolecarboxamide of the formula I as claimed in claim 1 and inert additives.

9. A method for controlling undesirable plant growth, wherein the plants or their habitat is or are treated with a herbicidal amount of an isoxazolecarboxamide of the formula I as claimed in claim 1.

10. The use of an isoxazolecarboxamide of the formula I as claimed in claim 1 as a herbicide.

## Revendications

1. Isoxazolecarboxamides répondant à la formule I 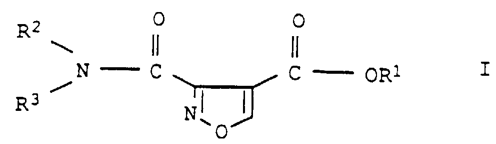 dans laquelle les symboles ont les significations suivantes :
R¹
(a) un groupe alkyle en C1-C6 qui peut porter un à cinq atomes d'halogènes et/ou jusqu'à trois groupes hydroxy et/ou alcoxy en C1-C4 et/ou l'un des substituants suivants :
- cyano ;
- (alcoxy en C1-C4)alcoxy en C2-C4 ;
- alkylthio en C1-C3 ;
- alkylamino en C1-C3, di-(alkyle en C1-C3)amine ;
- cycloalkylamino en C3-C6 ou di-(cycloalkyle en C3-C6)-amino ;
- tri-(alkyle en C1-C4)silyle ;
- carboxyle, (alcoxy en C1-C3)carbonyle ou (alcoxy en C1-C3)carbonyl-alcoxy en C1-C3, di-(alkyle en C1-C3)aminocarbonyle ;
- alcaniminoxy en C1-C6 ou cycloalcaniminoxy en C5-C6 ;
- un radical hétérocyclique à cinq ou six chaînons, saturé ou insaturé, contenant jusqu'à trois hétéroatomes choisis parmi l'oxygène, le soufre et l'azote, sous réserve que deux atomes d'oxygène et/ou de soufre ne peuvent pas être directement voisins, les hétérocycles pouvant encore porter un ou deux des substituants suivants : halogéno, alkyle en C1-C3 ou alcoxy en C1-C3 ;
- cycloalkyle en C3-C6 ;
- phényle, qui peut encore porter jusqu'à trois des substituants suivants : halogéno, nitro, cyano, alkyle en C1-C3, alkyle en C1-C3 partiellement ou totalement halogéné, alcoxy en C1-C3 ou alcoxy en C1-C3 partiellement ou totalement halogéné ;
- un groupe -CR¹⁰=N-R¹¹ dans lequel R¹⁰ et R¹¹ ont les significations suivantes :
R¹⁰ : hydrogène ou alkyle en C1-C6 et
R¹¹ : alcoxy en C1-C6, alcényloxy en C3-C6 ou alcynyloxy en C3-C6, chacun d'eux pouvant porter jusqu'à trois atomes d'halogènes et/ou un groupe phényle qui peut lui-même porter, si on le désire, jusqu'à trois des substituants suivants : halogéno, nitro, cyano, alkyle en C1-C3 ou alcoxy en C1-C3 ; alkylamino en C1-C6, di-(alkyle en C1-C6)amino ou phénylamino dans lequel le groupe phényle peut en outre porter jusqu'à trois des substituants suivants : halogéno, nitro, cyano, alkyle en C1-C3 ou alcoxy en C1-C3 ;
(b) cycloalkyle en C3-C8 qui peut porter un à cinq des substitùants suivants : halogéno, alkyle en C1-C4, alcoxy en C1-C4, alkyle en C1-C4 partiellement ou totalement halogéné ou alcoxy en C1-C4 partiellement ou totalement halogéné ;
(c) alcényle en C3-C6 à chaîne droite ou ramifiée, éventuellement substitué par des groupes alcynyle en C3-C6, des halogènes, des groupes alcoxy en C1-C4 ou le groupe phényle,
(d) cycloalcényle en C5-C6,
(e) alcynyle en C3-C6 à chaîne droite ou ramifiée éventuellement substitué par des halogènes, des groupes alcoxy en C1-C4 ou le groupe phényle, ce dernier pouvant lui-même porter un à trois des substituants suivants : halogéno, nitro, cyano, alkyle en C1-C4, alkyle en C1-C4 partiellement ou totalement halogéné ou alcoxy en C1-C4, alcoxy en C1-C4 partiellement ou totalement halogéné ;
(f) phényle, qui peut porter un à trois des substituants suivants : halogéno, nitro, cyano, alkyle en C1-C4, alkyle en C1-C4 partiellement ou totalement halogéné, alcoxy en C1-C4 ou alcoxy en C1-C4 partiellement ou totalement halogéné ;
(g) N-phtalimido, tétrahydrophtalimido, N-succinimido ou maléimido ;
R²
hydrogène ou alkyle en C1-C6 ;
R³
(a) un groupe alkyle en C₁-C₆ qui peut porter un à trois des substituants suivants : halogéno, cyano, alcoxy en C1-C4, cycloalkyle en C3-C8, phényle, le cycle phényle pouvant lui-même porter un à trois des substituants suivants : halogéno, alkyle en C1-C4, alkyle en C1-C4 partiellement ou totalement halogéné, alcoxy en C1-C4, alcoxy en C1-C4 partiellement ou totalement halogéné,
(b) un groupe cycloalkyle en C3-C8 qui peut porter un à trois des substituants suivants : halogéno, alkyle en C1-C6, alkyle en C1-C6 partiellement ou totalement halogéno, alcoxy en C1-C4 ou alcoxy en C1-C4 partiellement ou totalement halogéné ;
(c) un groupe alcényle à chaîne droite ou ramifiée en C3-C8 ou un groupe alcynyle à chaîne droite ou ramifiée en C3-C8, chacun d'eux pouvant porter un à trois substituants halogéno et/ou un substituant phényle, ce dernier pouvant lui-même porter un à trois des substituants suivants : alkyle en C1-C4, halogénoalcoxy en C1-C4, alcoxy en C1-C4, halogénoalcoxy en C1-C4, alkylthio en C1-C4, halogénoalkylthio en C1-C4, halogéno, cyano ou nitro ;
(d) un groupe phényle qui peut porter un à quatre des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, nitro, cyano, formyle, alcanoyle en C1-C4, halogénoalcanoyle en C1-C4 ou (alcoxy en C1-C4)carbonyle ;
ainsi que les sels des composés pour lesquels R² représente l'hydrogène qui sont bien tolérés par les végétaux.

2. Isoxazolecarboxamides de formule I selon la revendication 1 dans laquelle R¹ représente un groupe alkyle en C1-C6 ou cycloalkyle en C5-C6.

3. Isoxazolecarboxamides de formule I selon la revendication 1 dans laquelle R² représente l'hydrogène.

4. Isoxazolecarboxamides de formule I selon la revendication 1 dans laquelle R³ représente un groupe alkyle en C1-C6.

5. Isoxazolecarboxamides de formule I selon la revendication 1 dans laquelle R¹ représente un groupe alkyle en C1-C6 ou cycloalkyle en C5-C6, R³ un groupe alkyle en C1-C6 et R² l'hydrogène.

6. Procédé de préparation des isoxazolecarboxamides de formule-I' 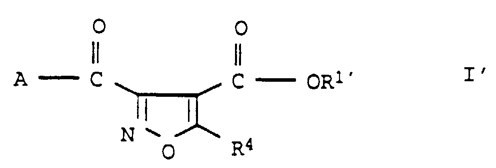 dans laquelle les symboles ont les significations suivantes :
R^{1'} : un groupe alkyle éventuellement substitué, cycloalkyle, phényle, alcényle ou alcynyle ;
A un groupe NR^{2'}R^{3'} ou un groupe OR⁵ ;
R^{2'} représente l'hydrogène ou un groupe alkyle ;
R^{3'} représente un groupe alkyle éventuellement substitué, cycloalkyle, alcényle, alcinyle ou aryle
ou bien R^{2'} et R^{3'} forment ensemble une chaîne CH₂CH₂CH₂CH₂- ou -CH₂CH₂CH₂CH₂CH₂-, dans laquelle un groupe CH₂ peut être remplacé par l'oxygène, le soufre ou le groupe NCH₃ ;
R⁵ représente un groupe alkyle éventuellement substitué, cycloalkyle, phényle, alcényle ou alcynyle ;
R⁴ représente l'hydrogène ou un groupe alkyle,
caractérisé par le fait que l'on fait réagir une énamide de formule II 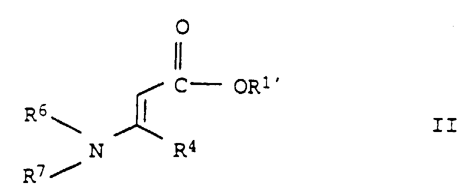 dans laquelle R⁶ et R⁷ représentent l'hydrogène ou des groupes alkyle en C1-C4 ou forment ensemble une chaîne -CH₂CH₂CH₂CH₂- ou -CH₂CH₂CH₂CH₂CH₂-, dans laquelle un groupe CH₂ peut être remplacé par l'oxygène, le soufre ou NCH₃, avec un chlorure d'acide hydroxamique de formule III

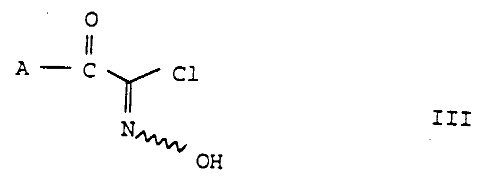

7. Procédé de préparation des isoxazolecarboxamides de formule I selon la revendication 1 caractérisé par le fait que l'on scinde un ester 4-alkylique d'isoxazole-3-carboxamide, de manière connue en soi, en présence d'une base, en l'acide IV 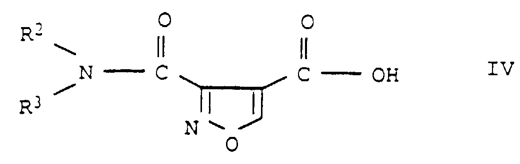 qu'on convertit de manière connue en soi, par estérification à l'aide d'un alcool de formule V
HOR¹ V
en le composé I.

8. Produits herbicides contenant un isoxazolecarboxamide de formule I de la revendication 1 et des additifs inertes.

9. Procédé pour combattre les croissances de végétaux indésirables, caractérisé par le fait que l'on traite les végétaux et/ou leur habitat par une quantité herbicide efficace d'un isoxazolecarboxamide de formule I selon la revendication 1.

10. Utilisation des isoxazolecarboxamides de formule I selon la revendication 1 en tant qu'herbicides.
